# EUROPEAN PATENT SPECIFICATION

(11) **EP 1 288 289 B1**
(45) Date of publication and mention of the grant of the patent: **18.02.2004**
(21) Application number: 01934356.5
(22) Date of filing: 25.05.2001
(51) Int. Cl.: C12N 1/00, C07H 3/02

(54) **NOVEL SACCHARIDE, PROCESS FOR PREPARATION OF THE SAME AND USE THEREOF**
NEUARTIGES SACCHARID, VERFAHREN ZU DESSEN HERSTELLUNG UND ANWENDUNG DESSELBEN
NOUVEAU SACCHARIDE, ET PROCEDE DE PREPARATION ET D'UTILISATION ASSOCIES

(30) Priority: 26.05.2000 JP 2000156673; 26.05.2000 JP 2000156674
(43) Date of publication of application: 05.03.2003
(73) Proprietor: Nihon Starch Co., Ltd., Kagoshim-shi, Kagoshima 891-0122 (JP); Hizukuri, Tomiko, Kagoshima-shi, Kagoshima (JP); Hizukuri, Kazuko, Kagoshima-shi Kagoshima (JP); Hizukuri, Kaoru, Chiba-shi, Chiba (JP); Abe, Junichi, Kagoshima-shi, Kagoshima 891-0145 (JP)
(72) Inventor: HIZUKURI, Susumu, Kagoshima-shi, Kagoshima 892-0811 (JP); ABE, Junichi, Kagoshima-shi, Kagoshima 891-0145 (JP); MUROYA, Kenkou, c/o Nihon Starch Co., Ltd., Kagoshima-shi, Kagoshima 891-0122 (JP); YOSHINAGA, Kazuhiro, c/o Nihon Starch Co., Ltd., Kagoshima-shi, Kagoshima 891-0122 (JP); FUJISUE, Mami, c/o Nihon Starch Co., Ltd., Kagoshima-shi, Kagoshima 891-0122 (JP); ISHIBA, Hideto, c/o Nihon Starch Co., Ltd., Kagoshima-shi, Kagoshima 891-0122 (JP)
(74) Representative: Cresswell, Thomas Anthony
(86) International application number: PCT/JP2001/004416
(87) International publication number: WO 2001/090299

(56) References cited:
- BERT F. REID ET AL.: 'Carbohydrates to densely functionalized carbocycles: Armed and disarmed effects in an approach to tetrodotoxin' PURE & APPL. CHEM. vol. 70, no. 2, 1998, pages 285 - 288, XP002945462
- HOWARD A.J. CARLESS: 'Complementry enantiospecific syntheses of conduritol E epoxides from halobenzanes' TETRAHEDRON LETTERS vol. 33, no. 42, 1992, pages 6379 - 6382, XP002945463
- EUGENE S. STEVENS: 'Calculation of the optical rotation of some anhydro sugars in solution' CARBOHYDRATE RESEARCH vol. 244, 1993, pages 191 - 195, XP002945464

## Description

### Technical Field

The present invention relates to a novel carbohydrate, its production method, and its use as an antioxidant.

### Background Art

An antioxidant has an effect of preventing deterioration of food and an effect of eliminating active oxygen which causes an adverse effect in a living body and is widely used in such applications as food, drugs and cosmetics. As a commercially available antioxidant, various antioxidants such as a chemical synthesis and an extract from a natural material are produced and sold. The antioxidants are roughly divided into two types, i.e., an oil-soluble antioxidant and a water-soluble antioxidant. As the oil-soluble antioxidant, a vitamin E, BHA and BHT are well-known. However, the vitamin E has a problem of very high production cost since the vitamin E is contained in a plant in a very small amount and the very small amount of the vitamin E must be extracted. As for the BHA and BHT which are chemical syntheses, their safety is recently concerned and their consumptions are on the decrease. Meanwhile, as the water-soluble antioxidant, a vitamin C is best-known and is widely used in such applications as food and drugs. Further, it is recently proposed to use, as an antioxidant, 1,5-D-anhydrofructose which is a saccharide obtained through decomposition of a starch by an enzyme (JP-A 9-505988).

### Disclosure of the Invention

An object of the present invention is to provide a novel carbohydrate having an antioxidation ability.

Another object of the present invention is to provide a novel carbohydrate having an antioxidation ability which is produced from 1,5-D-anhydrofructose.

Still another object of the present invention is to provide use of the novel carbohydrate of the present invention as an antioxidant.

Other objects and advantages of the present invention will be apparent from the following description.

According to the present invention, firstly, the above objects and advantages of the present invention are achieved by a novel carbohydrate:
(a) having neither a carbon-carbon double bond nor a carbonyl group and represented by a molecular formula C₆H₁₀O₅,
(b) showing a retention time of 11.0 ± 0.2 minutes when maltose and fructose as reference materials show retention times of 8.8 minutes and 12.5 minutes, respectively, the retention times being measured by high performance liquid chromatography using a column having, as a filler, a strong acid ion exchange resin which is a styrene-divinylbenzene copolymer with a degree of crosslinkage of 8% and has a sulfonic group bonded to sodium as an ion exchange group, at a column temperature of 40°C and a flow rate of water as an eluent of 1 ml/min, and
(c) showing an intramolecular carbon chemical shift value obtained by carbon nuclear magnetic resonance of 70.54 ± 1 ppm, 95.37 ± 1 ppm, 75.09 ± 1 ppm, 68.86 ± 1 ppm, 77.99 ± 1 ppm and 64.15 ± 1 ppm.

Secondly, the above objects and advantages of the present invention are achieved by use of the novel carbohydrate of the present invention as an antioxidant.

### Brief Description of the Drawings

Fig. 1 is a high performance liquid chromatogram of a product obtained by the production method of the novel carbohydrate of the present invention which is described in the present specification.

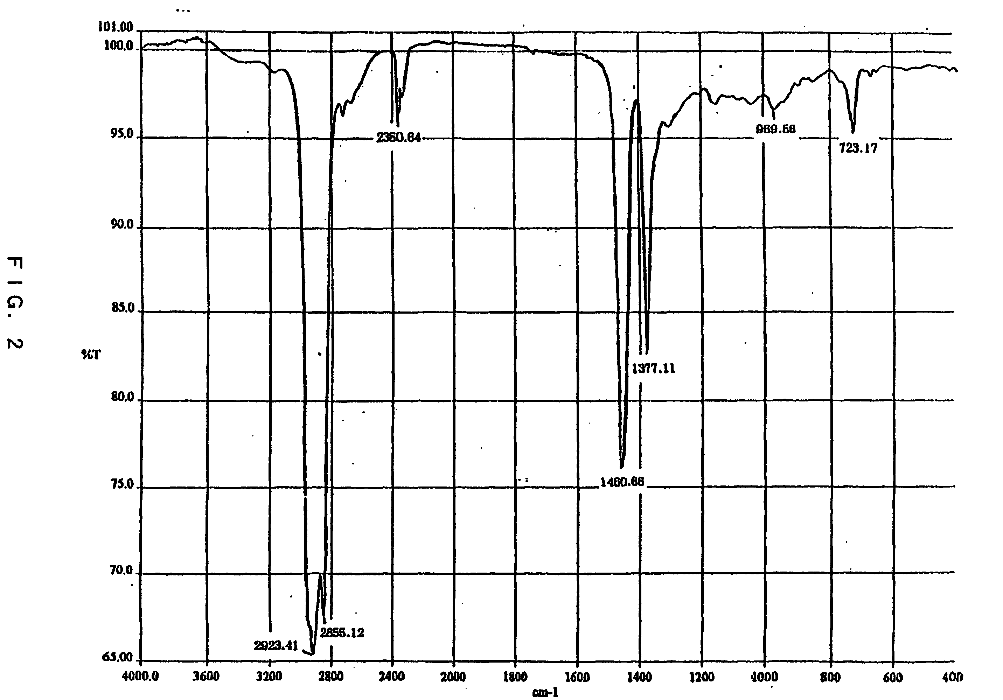

Fig. 2 is a diagram showing an infrared absorption spectrum of the novel carbohydrate of the present invention.

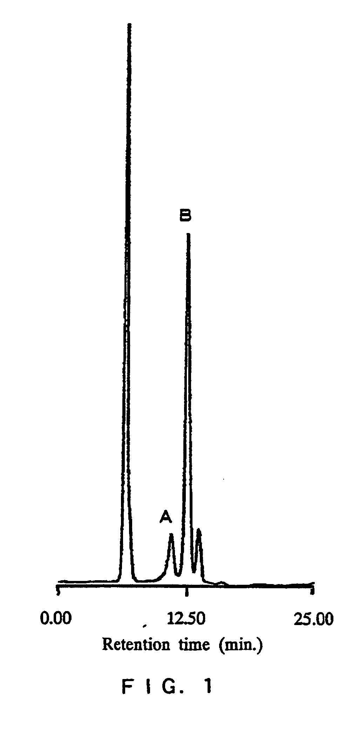

As for symbols in Fig. 1, A represents the novel carbohydrate of the present invention, and B represents 1,5-D-anhydrofructose.

### Best Mode for Carrying out the Invention

Hereinafter, the present invention will be described in detail.

The novel carbohydrate of the present invention can be produced by heating a solution prepared by dissolving 1,5-D-anhydrofructose in a 50-mM phosphoric acid buffer solution (pH: 8.0) in a concentration of 10 mg/ml, at 35°C for 5 hours.

The result of analyzing the solution heated by the foregoing method by high performance liquid chromatography using a column having, as a carrier, a strongly acidic cation exchange resin (MITSUBISHI CHEMICAL MCI GEL CK08S) which is a styrene-divinylbenzene copolymer with a degree of crosslinkage of 8% and has a sulfonic group bonded to sodium as an ion exchange group at a column temperature of 40° C and a flow rate of water as an eluent of 1 ml/min and using a differential refractometer as a detector is shown in Table 1. Further, at that time, maltose and fructose as reference materials showed 8.8 minutes and 12.5 minutes, respectively.

Then, by use of its high performance liquid chromatograph, the solution was purified repeatedly by fractionating so as to eventually obtain a novel carbohydrate of 99% or higher degree of purification. The material was obtained as white powders by freeze-drying.

Properties of the material were measured by high performance liquid chromatography, nuclear magnetic resonance spectroscopy, mass spectrometry (FAB-MS) and infrared absorption spectroscopy. The high performance liquid chromatography was carried out by use of MITSUBISHI CHEMICAL MCI GEL CK08S at a flow rate of water as an eluent of 1 ml/min and a column temperature of 40°C and by use of a differential refractometer as a detector. Under the conditions, the material showed a retention time of 11.0 ± 0.2 minutes. Further, at that time, maltose and fructose as reference materials showed 8.8 minutes and 12.5 minutes, respectively. Then, a nuclear magnetic resonance spectrum was measured.

The novel material was dissolved in heavy water in a concentration of 10%, and its nuclear magnetic resonance spectrum was recorded at 35° C by use of JEOL GSX-500 spectrum meter. As for chemical shifts, using 1,4-dioxane (67.4 ppm) as an external standard, chemical shifts from tetramethylsilane as an internal standard were expressed in ppm. As for attribution of signals, all signals could be attributed by measuring a two-dimensional nuclear magnetic resonance spectrum (¹H-¹H COSY, ¹H-¹³C COSY). The results are shown in Table 1.

**Table 1**

| Attribution | ¹H-NMR | | | ¹³C-NMR |
|---|---|---|---|---|
| | δ (ppm) | Multiplicity | J (Hz) | δ (ppm) |
| 1 | 3.532 | dd | J₁₁:11.75 | 70.543 |
| | 3.642 | d | J₁₃:1.28 | |
| 2 | | | | 95.367 |
| 3 | 3.822 | dd | J₃₄:3.21 | 75.091 |
| 4 | 3.763 | dd | J₄₅:10.04 | 68.864 |
| 5 | 3.583 | m | J₆₈:2.35, | 77.988 |
| | | | J₅₆:6.41 | |
| 6 | 3.666 | dd | J₆₆:12.18 | 64.152 |
| | 3.886 | dd | | |
| d: doublet, dd: doublet of doublet, m: multiplet | | | | |

Then, FAB-MS was measured in a negative mode by use of JEOL JMS-DX303 mass spectrometer. As a result, distinct peaks were observed with respect to molecular weights of 161 (162 - 1) and 323 (162 x 2 - 1). Thus, the molecular weight of the molecule was determined to be 162. Since this molecular weight corresponded to the molecular weight of hydrated anhydrofructose from which one water molecule had been dehydrated, it was found that the material had a molecular formula C₆H₁₀O₅.

Then, an infrared absorption spectrum was measured by use of Perkin Elmer FT-IR spectrum meter SPECTRUM 2000. The result is shown in Table 2. It is obvious from this result as well as the result of measurement of the nuclear magnetic resonance spectrum that neither a double bond nor a carbonyl group is present in the molecule.

Meanwhile, the 1,5-D-anhydrofructose used to produce the novel carbohydrate of the present invention can be produced by causing glucanlyase extracted and purified from red algae to act upon a waxy corn starch and then purifying the resulting product by gel filtration chromatography. That is, when the thus produced 1,5-D-anhydrofructose was identified by nuclear magnetic resonance spectroscopy, all carbon chemical shift values of the 1,5-D-anhydrofructose matched those of already reported 1,5-D-anhydrofructose. Further, the thus produced 1,5-D-anhydrofructose had a purity measured by high performance liquid chromatography of about 99%.

The novel carbohydrate of the present invention has a strong antioxidation ability.

As a measure of the antioxidation activity of the novel carbohydrate, active oxygen elimination activity was measured. The active oxygen elimination activity was measured by a nitrous acid kit method. This method is a method comprising the steps of producing a predetermined amount of active oxygen in the presence of enzyme, causing the active oxygen to react with the carbohydrate (antioxidant material) of the present invention and quantifying the amount of residual active oxygen so as to determine the activity of the carbohydrate. More specifically, in the nitrous acid kit method, a test liquid containing the carbohydrate of the present invention is added to a mixed solution of hydroxylamine and xanthine, and then a xanthine oxidase is added thereto so as to carry out a reaction of producing active oxygen by the xanthine oxidase and eliminating the active oxygen by the test liquid at 37°C for 30 minutes. Then, a coloring agent comprising naphthyl ethylene diamine is added to the mixture which is then left to stand at room temperature for 30 minutes, and residual active oxygen is then quantified by measuring absorbance at 550 nm. The active oxygen elimination activity of the carbohydrate of the present invention was compared with that of 1,5-D-anhydrofructose.

As a result, it was found that the active oxygen elimination activity of the novel carbohydrate of the present invention was 150 times that of the 1,5-D-anhydrofructose and that the novel carbohydrate was a material having a much higher antioxidation activity than the 1,5-D-anhydrofructose.

The novel carbohydrate obtained by the present invention can be used, as an antioxidant, in a food industry, a drug industry, a chemical industry, and other industries. As a method of using the novel carbohydrate, for example, the antioxidant of the present invention can be added to raw materials of food or a product. As another method, it is also possible that 1,5-D-anhydrofructose is added to a product and the method of the present invention is carried out during a food manufacturing process. For example, during the food manufacturing process, there often exists a heating step such as cooking or sterilization. Therefore, 1,5-D-anhydrofructose is added to raw materials of food in advance and the antioxidant material is produced in the production step such as cooking or sterilization in accordance with the method of the present invention. Further, in the case of alkaline food, if a formulation containing 1,5-D-anhydrofructose is added in advance, the antioxidant material is produced in accordance with the method of the present invention even during storage of the food.

## Claims

1. A carbohydrate:
(a) having neither a carbon-carbon double bond nor a carbonyl group and represented by a molecular formula C₆H₁₀O₅,
(b) showing a retention time of 11.0 ± 0.2 minutes when maltose and fructose as reference materials show retention times of 8.8 minutes and 12.5 minutes, respectively, the retention times being measured by high performance liquid chromatography using a column having, as a filler, a strong acid ion exchange resin which is a styrene-divinylbenzene copolymer with a degree of crosslinkage of 8% and has a sulfornic group bonded to sodium as an ion exchange group, at a column temperature of 40°C and a flow rate of water as an eluent of 1 ml/min, and
(c) showing intramolecular carbon chemical shift values obtained by carbon nuclear magnetic resonance of 70.54 ± 1 ppm, 95.37 ± 1 ppm, 75.09 ± 1 ppm, 68.86 ± 1 ppm, 77.99 ± 1 ppm and 64.15 ± 1 ppm.

2. Use of the carbohydrate of claim 1 as an antioxidant.

## Patentansprüche

1. Ein Kohlenhydrat, welches
(a) weder eine Kohlenstoff-Kohlenstoff-Doppelbindung noch eine Carbonylgruppe aufweist und durch eine Molekularformel C₆H₁₀O₅ dargestellt wird,
(b) eine Retentionszeit von 11,0 ± 0,2 Minuten zeigt, wenn Maltose und Fructose als Referenzmaterialien Retentionszeiten von jeweils 8,8 Minuten und 12,5 Minuten zeigen, wobei die Retentionszeiten mittels Hochleistungs-Flüssigkeitschromatographie gemessen werden, wobei eine Säule mit einem stark saurem Ionenaustauschharz, das ein Styrol-Divinylbenzol-Copolymer mit einem Vernetzungsgrad von 8% ist und als Ionenaustauschgruppe eine an Natrium gebundene Sulfongruppe aufweist, als Füllstoff verwendet wird, bei einer Säulentemperatur von 40°C und einer Fließgeschwindigkeit von Wasser als Elutionsmittel von 1 ml/min, und
(c) intramolekulare chemische Verschiebungswerte von Kohlenstoff, erhalten mittels Kohlenstoff-magnetische Kemresonanzspektroskopie, von 70,54 ± 1 ppm, 95,37 ± 1 ppm, 75,09 ± 1 ppm, 68,86 ± 1 ppm, 77,99 ± 1 ppm und 64,15 ± 1 ppm, aufweist.

2. Verwendung eines Kohlenhydrats gemäß Anspruch 1 als Antioxidationsmittel.

## Revendications

1. Hydrate de carbone :
(a) n'ayant ni double liaison carbone-carbone ni groupe carbonyle, et représenté par la formule brute C₆H₁₀O₅,
(b) présentant un temps de rétention de 11, 0 ± 0, 2 minutes alors que le maltose et le fructose pris comme produits de référence présentent respectivement des temps de rétention de 8, 8 minutes et 12, 5 minutes, les temps de rétention étant déterminés par chromatographie en phase liquide à haute performance, sur une colonne contenant une résine échangeuse d'ions, acide fort, qui est un copolymère de styrène et de divinylbenzène, ayant un degré de réticulation de 8 % et possédant des groupes sulfoniques liés à du sodium en tant que groupes échangeurs d'ions, à une température de colonne de 40 °C et un débit d'eau comme éluant de 1 ml/minute, et
(c) présentant des valeurs de déplacement chimique du carbone intramoléculaire, obtenues par résonance magnétique nucléaire du carbone, de 70, 54 ± 1 ppm, 95, 37 ± 1 ppm, 75, 09 ± 1 ppm, 68, 86 ± 1 ppm, 77, 99 ± 1 ppm et 64, 15 ± 1 ppm.

2. Utilisation de l'hydrate de carbone selon la revendication 1, comme antioxydant.
